# EUROPEAN PATENT SPECIFICATION

(11) **EP 4 247 541 B1**
(45) Date of publication and mention of the grant of the patent: **28.08.2024**
(21) Application number: 21840224.6
(22) Date of filing: 23.11.2021
(51) Int. Cl.: B01J 19/00, C07K 1/04, C12N 9/16

(54) **METHODS OF MAKING AND USING PLATFORMS FOR PEPTIDE SYNTHESIS AND COMPOSITIONS THEREOF**
VERFAHREN ZUR HERSTELLUNG UND VERWENDUNG VON PLATTFORMEN ZUR PEPTIDSYNTHESE UND ZUSAMMENSETZUNGEN DAVON
PROCÉDÉS DE FABRICATION ET D'UTILISATION DE PLATEFORMES POUR LA SYNTHÈSE DE PEPTIDES ET COMPOSITIONS ASSOCIÉES

(30) Priority: 23.11.2020 US 202063117190 P
(43) Date of publication of application: 27.09.2023
(73) Proprietor: Peptilogics, Inc., Pittsburgh, PA 15203 (US)
(72) Inventor: LEE, Francis, Pittsburgh, Pennsylvania 15203 (US)
(74) Representative: Gille Hrabal Partnerschaftsgesellschaft mbB Patentanwälte
(86) International application number: PCT/US2021/060515
(87) International publication number: WO 2022/109456

(56) References cited:
- WO-A1-2013/057186
- WO-A1-2020/201450
- US-A1- 2007 141 724
- US-B1- 6 600 016

## Description

### TECHNICAL FIELD

The present invention relates to methods of making and using platforms for peptide synthesis and compositions thereof, such as peptide-anchored resins or beads for use in solid-phase peptide synthesis.

### BACKGROUND

Peptide synthesis is the production of peptides, which are compounds in which multiple amino acids are linked via amide bonds (also known as peptide bonds). Peptides can be chemically synthesized by the condensation reaction of the carboxyl group of one amino acid to the amino group of another.

The chemical synthesis of peptides can be carried out using classical solution-phase techniques, although these have been replaced in most research and development settings by solid-phase methods. Solid-phase peptide synthesis (SPPS) is a process used to chemically synthesize peptides on solid supports. A "solid support" is an insoluble polymeric support that is a solid-phase material or resin (most commonly, low cross-linked polystyrene beads).

SPPS allows the rapid assembly of a peptide chain through successive reactions of amino acid derivatives on an insoluble porous support. Generally, the solid support includes small, polymeric resin functionalized with reactive groups (such as amine or hydroxyl groups) that link to the nascent peptide chain. Since the peptide remains covalently attached to the support throughout the synthesis, excess reagents and side products can be removed by washing and filtration. This approach circumvents the comparatively time-consuming isolation of the product peptide from solution after each reaction step, which would otherwise be required when using conventional solution-phase synthesis.

Depending on the side chain and the protection strategy used for the particular peptide being synthesized, each amino acid to be coupled to the peptide chain N-terminus must be protected on its N-terminus and side chain using appropriate protecting groups, such as Boc (acid-labile) or Fmoc (base-labile).

The general SPPS procedure is one of repeated cycles of alternating N-terminal deprotection and coupling reactions. The resin can be washed between each step. First, an amino acid is coupled to the resin. Subsequently, the amine is deprotected, and then coupled with the free acid of the second amino acid. This cycle repeats until the desired sequence has been synthesized.

SPPS cycles may also include capping steps, which block the ends of unreacted amino acids from reacting. At the end of the synthesis, using a reagent strong acid like trifluoroacetic acid or a nucleophile, the crude peptide is cleaved from the solid support while simultaneously removing all protecting groups. To remove organic soluble by-products, the crude peptide can be precipitated from a non-polar solvent like diethyl ether. The crude peptide can be purified using reversed-phase HPLC. The purification process, especially of longer peptides, can be challenging, because small amounts of several byproducts, which are very similar to the product, have to be removed. For this reason, so-called continuous chromatography processes, such as MCSGP, are increasingly being used in commercial settings to maximize the yield without sacrificing on purity levels.

SPPS is limited by reaction yields, and typically peptides and proteins in the range of 70 amino acids are pushing the limits of synthetic accessibility. Future developments may lessen or extend this range, but the limitations of SPPS articulated herein shall apply within the context of whatever that range may be at any particular time. Synthetic difficulty also is sequence dependent; typically, aggregation-prone sequences (such as amyloids) are difficult to make. Longer lengths can be accessed by using ligation approaches such as native chemical ligation, where two shorter fully deprotected synthetic peptides can be joined together in solution.

The use of N-terminal and side chain protecting groups is essential during peptide synthesis to avoid undesirable side reactions, such as self-coupling of the activated amino acid leading to polymerization. This would compete with the intended peptide coupling reaction, resulting in low yield or even in complete failure to synthesize the desired peptide.

Two principle orthogonal protecting group schemes exist for use in solid-phase peptide synthesis: the so-called Boc/Bzl and Fmoc/tBu approaches. The Boc/Bzl strategy utilizes TFA-labile N-terminal Boc protection alongside side chain protection that is removed using anhydrous hydrogen fluoride during the final cleavage step (with simultaneous cleavage of the peptide from the solid support). Fmoc/tBu SPPS uses base-labile Fmoc N-terminal protection, with side chain protection and a resin linkage that are acid-labile (final acidic cleavage is carried out via trifluoroacetic acid (TFA) treatment).

The original method for peptide synthesis relied on tert-butyloxycarbonyl ("Boc") as a temporary N-terminal α-amino protecting group. The Boc group is removed with acid, such as TFA. This forms a positively charged amino group in the presence of excess TFA, which is neutralized and coupled to the incoming activated amino acid. Neutralization can either occur before coupling or *in situ* during the basic coupling reaction.

The Boc/Bzl approach retains its usefulness in reducing peptide aggregation during synthesis. In addition, Boc/Bzl SPPS may be preferred over the Fmoc/tBu approach when synthesizing peptides containing base-sensitive moieties (such as depsipeptides), as treatment with base is required during the Fmoc deprotection step.

Permanent side-chain protecting groups used during Boc/Bzl SPPS are typically benzyl or benzyl-based groups. Final removal of the peptide from the solid support using anhydrous hydrogen fluoride via hydrolytic cleavage occurs simultaneously with side chain deprotection. The final product is a fluoride salt which is relatively easy to solubilize. Scavengers such as cresol must be added to the HF to prevent reactive t-butyl cations from generating undesired products. A disadvantage of this approach is the potential for degradation of the peptide by hydrogen fluoride.

The use of N-terminal Fmoc protection allows for a milder deprotection scheme than used for Boc/Bzl SPPS, and this protection scheme is truly orthogonal under SPPS conditions. Fmoc deprotection utilizes a base, typically 20-50% piperidine in DMF. The exposed amine is therefore neutral, and consequently no neutralization of the peptide-resin is required, as in the case of the Boc/Bzl approach. The lack of electrostatic repulsion between the peptide chains can lead to increased risk of aggregation with Fmoc/tBu SPPS, however. Because the liberated fluorenyl group is a chromophore, Fmoc deprotection can be monitored by UV absorbance of the reaction mixture, a strategy employed in automated peptide synthesizers.

The ability of the Fmoc group to be cleaved under relatively mild basic conditions while being stable to acid allows the use of side chain protecting groups such as Boc and tBu that can be removed in milder acidic final cleavage conditions (TFA) than those used for final cleavage in Boc/Bzl SPPS (HF). Scavengers such as water and triisopropylsilane (TIPS) are added during the final cleavage to prevent side reactions with reactive cationic species released as a result of side chain deprotection. The resulting crude peptide is obtained as a TFA salt, which is potentially more difficult to solubilize than the fluoride salts generated in Boc SPPS.

Examples of systems for flow peptide synthesis are shown in **FIG 1** (Erickson's flow peptide synthesizer) and **FIG. 2** (Pentelute's automated fast-flow peptide synthesizer). **FIG. 3** is an overview of a representative SPPS peptide synthesis process (Fmoc approach).

The platform having the protecting groups is tailored to the particular synthesis process being performed. Thus, when the need arises for a different synthesis process to be performed, the system has the platform changed over for the different synthesis, which is timely, costly, and burdensome. Accordingly, there remains a disadvantage of this process in that the platform used for peptide synthesis has to be changed out and replaced depending upon the particular synthesis process employed.

Therefore, the need remains for a universal platform for peptide synthesis, which can then be utilized in the system for the synthesis of making different peptides (sequentially) without the need of replacing the platform. WO2013/057186 discloses a method for solid-state synthesis of peptides.

### SUMMARY OF THE INVENTION

The present invention relates to methods of making and using platforms for peptide synthesis and compositions thereof, such peptide-anchored resins or beads for use in solid-phase peptide synthesis.

In general, in one aspect, the invention features a method of making a platform for a solid-state peptide synthesis process. The method includes selecting an insoluble carrier material. The insoluble carrier material is in the form of a plurality of particles. The method further includes coupling a plurality of different linkers to each of the plurality of particles of the insoluble carrier material to form a plurality of platform particles of the platform. The plurality of different linkers include (a) Fmoc-2,4-dimethoxy-4'-(carboxymethyloxy)-benzhydrylamine (Rink amide linker); (b) 4-Formyl-3-methoxy-phenoxyacetic acid; (c) 2-Hydroxy-5-dibenzosuberone; (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10,11-dihydro-SH-dibenzo[a,d] cycloheptene).

Implementations of the invention can include one or more of the following features:
The insoluble carrier material can be a resin material.

The particles can be microparticles.

The particles can be nanoparticles.

The nanoparticles can have an average diameter of less than 100 nm.

The platform can be a universal platform for a solid-state peptide synthesis process.

In general, in another aspect, the invention features a method of making a platform for a solid-state peptide synthesis process. The method includes selecting an insoluble carrier material, wherein the insoluble carrier material is in the form of a plurality of particles. The method further includes coupling a plurality of different linkers to each of the plurality of particles of the insoluble carrier material to form a plurality of platform particles of the platform. The plurality of different linkers comprises at least three of the linkers selected from the group consisting of (a) Fmoc-2,4-dimethoxy-4'-(carboxymethyloxy)-benzhydrylamine (Rink amide linker); (b) 4-Formyl-3-methoxy-phenoxyacetic acid; (c) 2-Hydroxy-5-dibenzosuberone; (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10,11-dihydro-5H-dibenzo[a,d] cycloheptene).

Implementations of the invention can include one or more of the following features:
The plurality of different linkers can include (a) Fmoc-2,4-dimethoxy-4'-(carboxymethyloxy)-benzhydrylamine (Rink amide linker); (b) 4-Formyl-3-methoxy-phenoxyacetic acid; and (c) 2-Hydroxy-5-dibenzosuberone.

The plurality of different linkers can further include one or more of the linkers selected from the group consisting of: (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10,11-dihydro-5H-dibenzo[a,d]cycloheptene).

The insoluble carrier material can be a resin material.

The particles can be microparticles.

The particles can be nanoparticles.

The nanoparticles can have an average diameter of less than 100 nm.

The platform can be a universal platform for a solid-state peptide synthesis process.

In general, in another aspect, the invention features a platform composition capable of being used in a solid-state peptide synthesis process to synthesize peptides. The platform composition includes an insoluble carrier material. The insoluble carrier material is in the form of a plurality of particles. The platform composition further includes a plurality of different linkers. Each of the plurality of particles of the insoluble carrier material is coupled to at least one of each of the different linkers in the plurality of linkers. The plurality of different linkers include (a) Fmoc-2,4-dimethoxy-4'-(carboxymethyloxy)-benzhydrylamine (Rink amide linker); (b) 4-Formyl-3-methoxy-phenoxyacetic acid; (c) 2-Hydroxy-5-dibenzosuberone; (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10,11-dihydro-5H-dibenzo[a,d] cycloheptene).

Implementations of the invention can include one or more of the following features:
The insoluble carrier material can be a resin material.

The particles can be microparticles.

The particles can be nanoparticles.

The nanoparticles can have an average diameter of less than 100 nm.

The platform composition can be capable of being used as a universal platform in a solid-state peptide synthesis process to produce peptides.

In general, in another aspect, the invention features a platform composition capable of being used in a solid-state peptide synthesis process to synthesize peptides. The platform composition includes an insoluble carrier material. The insoluble carrier material is in the form of a plurality of particles. The platform composition further includes a plurality of different linkers. Each of the plurality of particles of the insoluble carrier material is coupled to at least one of each of the different linkers in the plurality of linkers. The plurality of different linkers includes at least three of the linkers selected from the group consisting of (a) Fmoc-2,4-dimethoxy-4'-(carboxymethyloxy)-benzhydrylamine (Rink amide linker); (b) 4-Formyl-3-methoxy-phenoxyacetic acid; (c) 2-Hydroxy-5-dibenzosuberone; (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10,11-dihydro-5H-dibenzo[a,d] cycloheptene).

Implementations of the invention can include one or more of the following features:
The plurality of different linkers can include (a) Fmoc-2,4-dimethoxy-4'-(carboxymethyloxy)-benzhydrylamine (Rink amide linker); (b) 4-Formyl-3-methoxy-phenoxyacetic acid; and (c) 2-Hydroxy-5-dibenzosuberone.

The plurality of different linkers can further include one or more of the linkers selected from the group consisting of: (d) 4-Hydroxymethylbenzoic acid (HMBA); (e) 4-Hydroxymethyl-phenoxyacetic acid (HMP linker); (f) 4-(Fmoc-hydrazino)-benzoic acid; (g) 4(4-(1-hydroxyethyl)-2-methoxy-5-nitrophenoxy)-butyric acid; and (h) Fmoc-Suberol (5-Fmoc-amino-2-carboxymethoxy-10, 11-dihydro-5H-dibenzo[a,d]cycloheptene).

The insoluble carrier material can be a resin material.

The particles can be microparticles.

The particles can be nanoparticles.

The nanoparticles can have an average diameter of less than 100 nm.

The platform can be a universal platform for a solid-state peptide synthesis process.

In general, in another aspect, the invention features a method that includes loading one of the above-described platform compositions into a reaction vessel of a solid-phase peptide synthesis synthesizer. The method further includes utilizing the solid-phase peptide synthesis synthesizer to synthesize a first peptide using the platform composition as the platform for peptide synthesis.

Implementations of the invention can include one or more of the following features:
The method can further include, after the step of synthesizing the first peptide, utilizing the solid-phase peptide synthesis synthesizer to synthesize a second peptide. The solid-state peptide synthesis synthesizer can use the platform composition as the platform for peptide synthesis. The second peptide can be a different peptide from the first peptide.

The solid-phase peptide synthesis synthesizer can be a continuous flow peptide synthesizer.

The method can further include measuring the flow in the continuous flow peptide synthesizer to quantitatively measure the synthesis of the first peptide.

### DESCRIPTION OF DRAWINGS

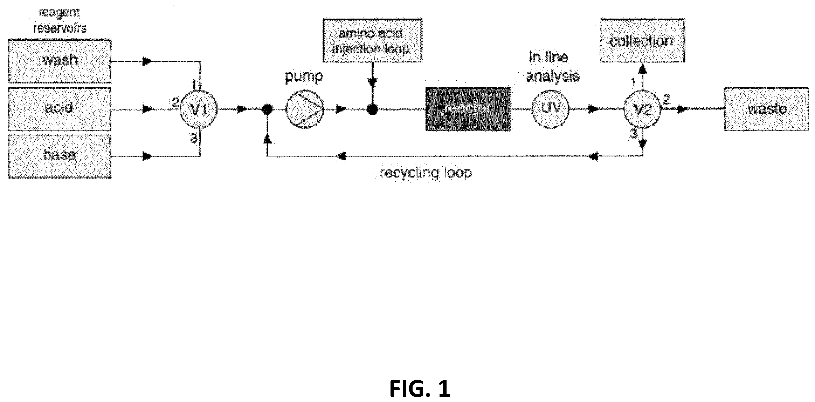

**FIG. 1** is a schematic of a flow peptide synthesis known in the prior art.

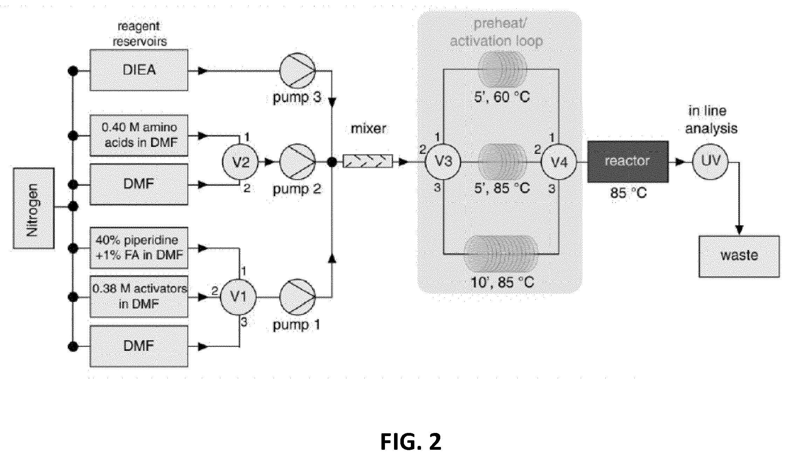

**FIG. 2** is a schematic of an automated fast-flow peptide synthesizer known in the prior art.

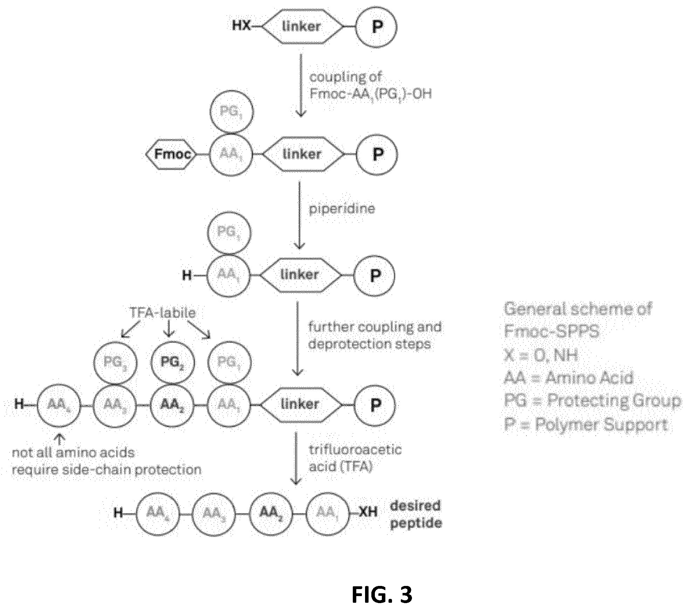

**FIG. 3** is an overview of a representative SPPS peptide synthesis process.

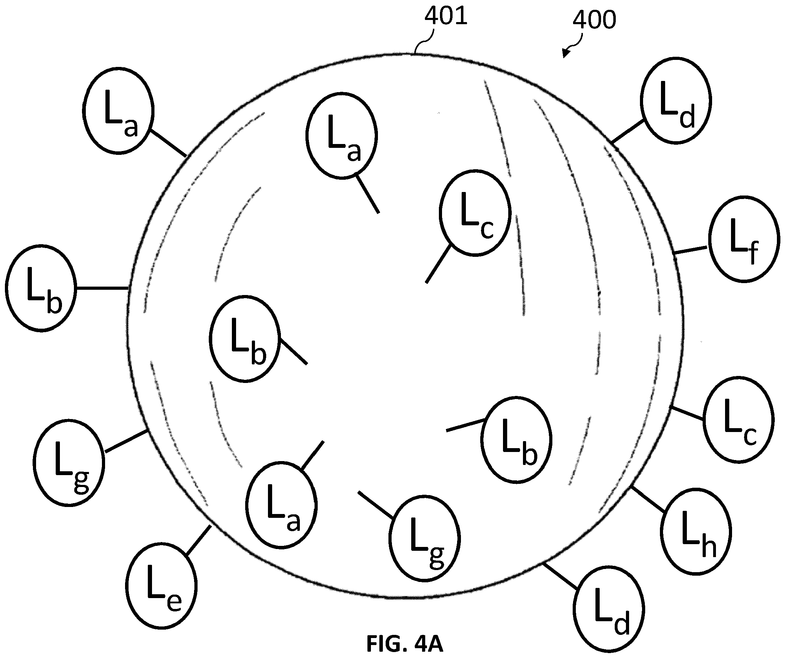

**FIG. 4A** is an illustration of a particle of the platform.

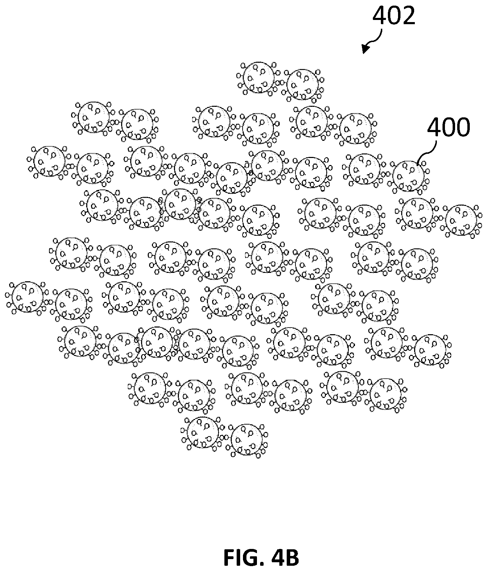

**FIG. 4B** is an illustration of a platform having a plurality of particles shown in **FIG.4A****.**

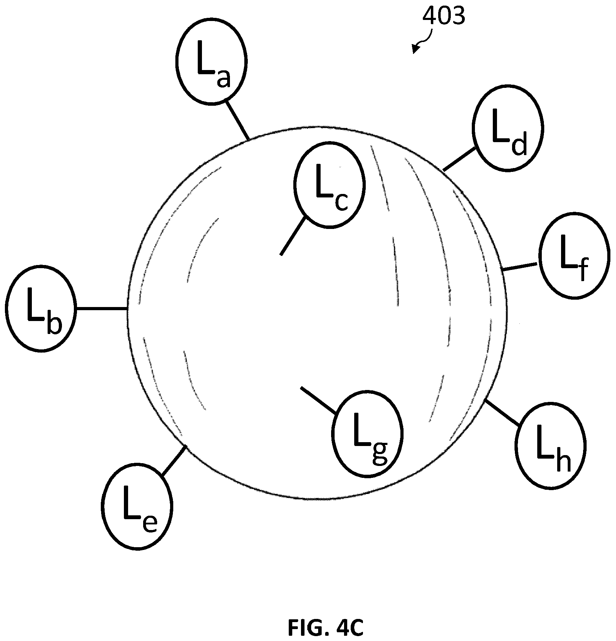

**FIG. 4C** is an illustration of an alternative particle of the platform.

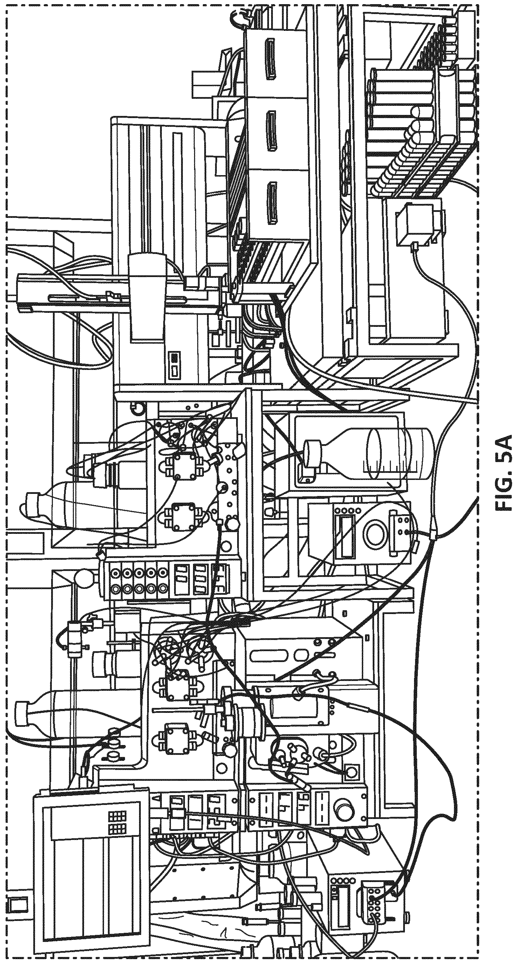

**FIG. 5A** is a photograph of a continuous flow SPPS setup in which embodiments of the platform of the present invention can be utilized.

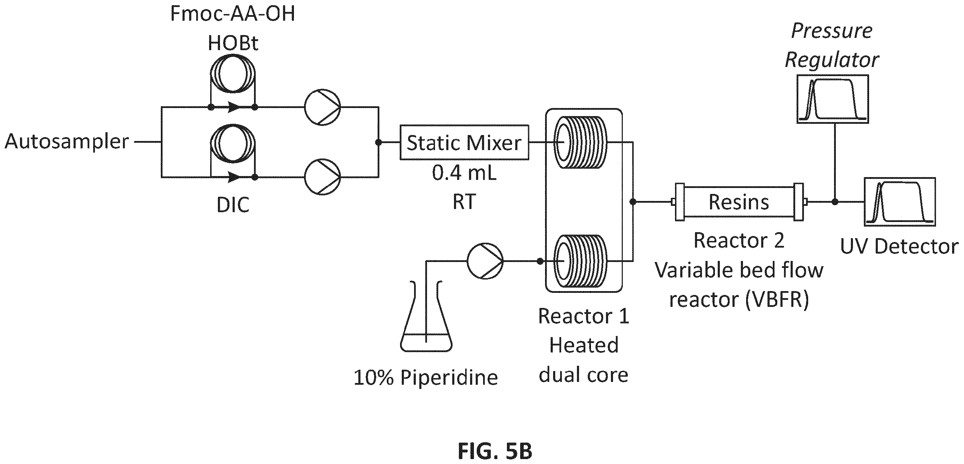

**FIG. 5B** is a schematic of the continuous flow SPPS setup shown in **FIG. 4A****.**

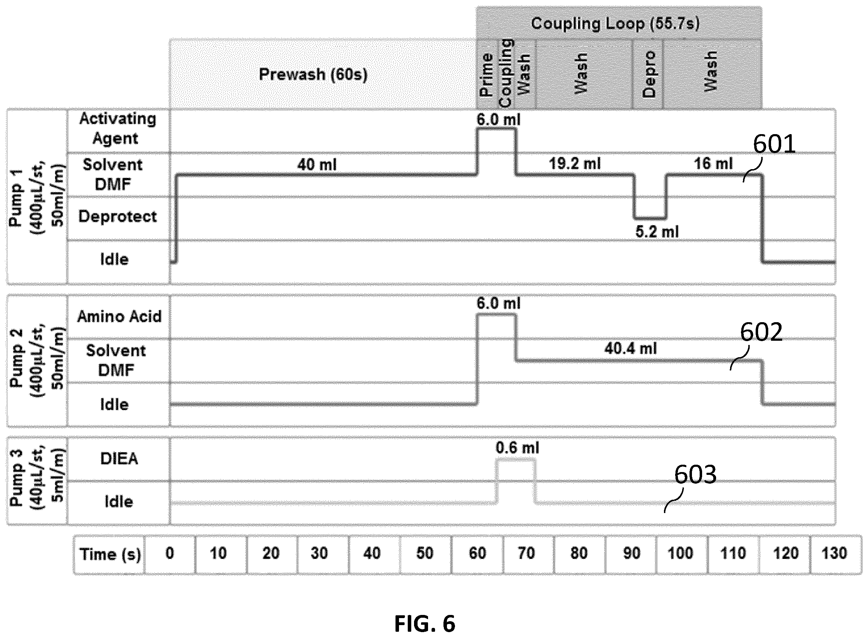

**FIG. 6** is a flow time diagram for an embodiment of the present invention.

### DETAILED DESCRIPTION

The present invention relates to methods of making and using platforms for peptide synthesis and compositions thereof, such peptide-anchored resins or beads for use in solid-phase peptide synthesis. The resins and beads are universal platforms in that these can be used in varieties of peptide synthesis, and do not need to be replaced in the system even when a different peptide synthesis is to be performed.

### Platform

A universal platform (in resin and bead form) has been discovered that is a universal platform for peptide synthesis. The platform is a carrier material made of particles, with each particle having a plurality of distinct types of linkers (otherwise referred to anchors). Generally, the particles are microparticle (or nanoparticle) in size and are generally of the same size (i.e., generally these are monosized with the majority within 20% of the average size of the particles)

The linkers are protective groups that are bifunctional molecules that anchor the growing peptide to the insoluble carrier particle. Due to this plurality of distinct types of linkers, the platform is universal in that it can be used for various SPPS processes without having to be replaced between the transition from the production of a first peptide to the production of a second (and distinct) peptide.

The present invention utilizes insoluble carrier material suitable for SPPS, which generally can be a resin material. While polystyrene-based resins are typically utilized in SPPS processes, other polymer resins can be utilized due to the coupling of the plurality of linkers.

In embodiments of the present invention, the plurality of linkers includes:
(a) Fmoc-2,4-dimethoxy-4'-(carboxymethyloxy)-benzhydrylamine (Rink amide linker);
(b) 4-Formyl-3-methoxy-phenoxyacetic acid;
(c) 2-Hydroxy-5-dibenzosuberone;
(d) 4-Hydroxymethylbenzoic acid (HMBA);
(e) 4-Hydroxymethyl-phenoxyacetic acid (HMP linker);
(f) 4-(Fmoc-hydrazino)-benzoic acid;
(g) 4(4-(1-hydroxyethyl)-2-methoxy-5-nitrophenoxy)-butyric acid; and
(h) Fmoc-Suberol (5-Fmoc-amino-2-carboxymethoxy-10,11-dihydro-5H-dibenzo[a,d] cycloheptene).

**FIG. 4A** illustrates a platform particle **400** that includes an insoluble carrier material particle **401** having a plurality of linkers (a)-(h) (which linkers (a)-(h) are designated as Lₐ-Lₕ, respectively). **FIG. 4B** is an illustration of a platform **402** that includes a plurality of the platform particles **400.** In such embodiment, each of the linkers (a)-(h) (Lₐ-Lₕ) of platform particle **400** is coupled to each of the insoluble carrier material particles **401.** For instance, the C-terminal Fmoc amino acid may be coupled to the linker yielding the so-called handle, which can be purified before loading the polymer. Regardless of the bulkiness of the amino acid, high loads are obtained by coupling these handles.

In other embodiments, a subset of three of more of linkers (a)-(h) is coupled to the insoluble carrier. For example, by coupling linkers (a)-(c) to the insoluble carrier, what would result is a viable platform for the synthesis of all naturally occurring peptides and the synthesis of about 20% of non-naturally occurring peptides. The term "universal" platform as used in this paragraph and elsewhere herein is not intended to mean that the platform is a viable platform for all SPPS processes; rather, it means that the platform is capable of being utilized in a variety of different SPPS processes to yield the majority of naturally occurring peptides.

In other embodiments, the subset of linkers includes linkers (a)-(c) plus one or more of linkers (d)-(h). And, in still further embodiments, the subset of linkers includes one or two of linkers (a)-(v) plus one or more of linkers (d)-(h).

The number of each of linkers (a)-(f) attached to an individual particle is generally more than one, *i.e.,* the individual particles have two or more of linker (a), two or more of linker (b), *etc.*

In some embodiments, such as shown in the platform particle **403** illustrated in **FIG. 4C****,** the individual particles have a one-to-one correspondence in that there is generally one of linker (a), one of linker (b), etc. coupled to the individual particle. In such instance, the individual particles can be nanoparticles, including nanoparticles having an average diameter of less than 100 nm.

### SPPS Method Utilizing The Platform

The platform of the present invention can then be utilized in an SPPS setup, such as the continuous flow SPPS setup shown in **FIGS. 5A-5B****.** For example, the platform as described above can be used for a first SPPS process in which the synthesis employs the Fmoc-2,4-dimethoxy-4'-(carboxymethyloxy)-benzhydrylamine (Rink amide linker) linker for the first amino group. Once this has occurred, the washing and deactivation step then precludes the other linkers from being involved in subsequent steps occurring in the process. Moreover, during this process, to prevent byproducts of deprotection and cleavage from reacting with the peptide and forming undesired impurities, scavengers can be added.

Once this first SPPS process is completed, the platform can be used for a second SPPS process in the setup without having to replace the platform. For example, the platform as described above can be used for a second SPPS process in which the synthesis employs the 2-Hydroxy-5-dibenzosuberone linker for the first amino group.

**FIG. 6** is a flow time diagram for a system or an SPPS process, which system uses three pumps (pumps one through three) and the representative SPPS process has pumping profiles **601-603,** respectively, that can be performed with the platform. As reflected in **FIG. 6****,** (a) pump one is pumping the activating agent, solvent (DMF), and deprotection reagent, at 50 ml/min; (b) pump two is pumping amino acid, solvent (DMF) at 50 ml/min; and (c) pump three is pumping DIEA at 5 ml/min. By way of example, the activating agent can be such as activators in DMF, such as 0.38 M actuators in DMF; the deprotection reagent can be 40% piperidine + 1% FA in DMF, and the amino acids can be 0.4 M amino acids in DMF.

**TABLE I** (below) reflects the various materials and approximate times that are utilized during the first SPPS process for the profile shown in **FIG. 6****.**

**TABLE I**

| **Step** | **Description** | **Time** | **Pump** | **Material** | **Flow Rate** |
|---|---|---|---|---|---|
| 1 | Prewash | 0s to 12s | None | | 0 ml/min |
| 2 | Prewash | 12s to 60s | 1 | Solvent | 50 ml/min |
| 3 | Coupling Loop - Prime | 60s to 64.2s | 1 | Activating Agent | 50 ml/min |
| | | | 2 | Amino Acid | 50 ml/min |
| 4 | Coupling Loop - Coupling | 64.2s to 67.2s | 1 | Activating Agent | 50 ml/min |
| | | | 2 | Amino Acid | 50 ml/min |
| | | | 3 | DIEA | 5 ml/min |
| 5 | Coupling Loop - Wash | 67.2s to 71.4s | 1 | Solvent | 50 ml/min |
| | | | 2 | Solvent | 50 ml/min |
| | | | 3 | DIEA | 5 ml/min |
| 6 | Coupling Loop - Wash | 71.4s to 90.2s | 1 | Solvent | 50 ml/min |
| | | | 2 | Solvent | 50 ml/min |
| 7 | Coupling Loop - Deprotection | 90.2s to 96.5s | 1 | Deprotect | 50 ml/min |
| | | | 2 | Solvent | 50 ml/min |
| 8 | Coupling Loop - Wash | 96.5s to 115.7s | 1 | Solvent | 50 ml/min |
| | | | 2 | Solvent | 50 ml/min |
| 9 | Post Coupling Loop | 115.7s to 125s | None | | 0 ml/min |

Without replacing the platform, a second SPPS can be performed. **TABLE II** (below) reflects the various materials and approximate times that can be utilized during the second SPPS process.

**TABLE II**

| **Step** | **Description** | **Time** | **Pump** | **Material** | **Flow Rate** |
|---|---|---|---|---|---|
| 1 | Wash | 0s to 90s | 1 | DMI | 2.5 ml/min |
| | | | 2 | DMI | 2.5 ml/min |
| 2 | Wash | 90s to 120s | 1 | DCM | 2.5 ml/min |
| | | | 2 | DCM | 2.5 ml/min |
| 3 | Deprotection | 120s to 234s | 1 | DCM | 2.5 ml/min |
| | | | 2 | Detritylation stock | 2.5 ml/min |
| 4 | Wash | 234s to 252s | 1 | DCM | 2.5 ml/min |
| | | | 2 | DCM | 2.5 ml/min |
| 5 | Neutralization | 252s to 264s | 1 | DCM | 2.5 ml/min |
| | | | 2 | Neutralization stock | 2.5 ml/min |
| 6 | Wash | 264s to 288s | 1 | DCM | 2.5 ml/min |
| | | | 2 | DCM | 2.5 ml/min |
| 7 | Wash | 288s to 318s | 1 | DMI | 2.5 ml/min |
| | | | 2 | DMI | 2.5 ml/min |
| 8 | Coupling | 318s to 323s | 1 | Coupling base stock | 2 ml/min |
| | | | 2 | Monomer stock | 2 ml/min |
| 9 | Wash | 323s to 330s | 1 | DMI | 2 ml/min |
| | | | 2 | DMI | 2 ml/min |
| 10 | Wash | 330s to 750s | 1 | DMI | 0.1 ml/min |
| | | | 2 | DMI | 0.1 ml/min |

Another advantage of the platform particles is that these can be quantitatively measured by flow, particularly for those particles that have a one-to-one correspondence of linker to particle (such as platform particle **403** illustrated in **FIG. 4C****).**

This present invention provides a flow chemistry advantage that will reduce the reagent consumption, have inherent automation potential, be suitable for real time/in-line monitoring, be more repeatable, have improved heat and mass transfer (mixing and heating), and be utilized at extreme reaction conditions (as there is more variation in the insoluble carrier material).

Additional advantages include that the platform has the added versatility in view of its being a universal platform, the ability to use the platform in scalable processes, and the ability to expand the peptides that can be synthesized with the same platform.

While embodiments of the invention have been shown and described, modifications thereof can be made by one skilled in the art without departing from the spirit and teachings of the invention. The embodiments described and the examples provided herein are exemplary only and are not intended to be limiting. Many variations and modifications of the invention disclosed herein are possible and are within the scope of the invention. The scope of protection is not limited by the description set out above, but is only limited by the claims which follow, that scope including all equivalents of the subject matter of the claims.

The disclosures of all patents, patent applications, and publications cited herein are hereby incorporated herein by reference in their entirety, to the extent that they provide exemplary, procedural, or other details supplementary to those set forth herein.

Amounts and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a numerical range of approximately 1 to approximately 4.5 should be interpreted to include not only the explicitly recited limits of 1 to approximately 4.5, but also to include individual numerals such as 2, 3, 4, and sub-ranges such as 1 to 3, 2 to 4, etc. The same principle applies to ranges reciting only one numerical value, such as "less than approximately 4.5," which should be interpreted to include all of the above-recited values and ranges. Further, such an interpretation should apply regardless of the breadth of the range or the characteristic being described.

Unless defined otherwise, all technical and scientific terms used herein have the same meaning as commonly understood to one of ordinary skill in the art to which the presently disclosed subject matter belongs. Although any methods, devices, and materials similar or equivalent to those described herein can be used in the practice or testing of the presently disclosed subject matter, representative methods, devices, and materials are now described.

Following long-standing patent law convention, the terms "a" and "an" mean "one or more" when used in this application, including the claims.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in this specification and attached claims are approximations that can vary depending upon the desired properties sought to be obtained by the presently disclosed subject matter.

As used herein, the term "about" and "substantially" when referring to a value or to an amount of mass, weight, time, volume, concentration or percentage is meant to encompass variations of the following: in some embodiments ±20%, in some embodiments ± 10%, in some embodiments ±5%, in some embodiments ±1%, in some embodiments ±0.5%, and in some embodiments ±0.1%, each with respect to or from the specified amount, as such variations are appropriate to perform the disclosed method.

As used herein, the term "and/or" when used in the context of a listing of entities, refers to the entities being present singly or in combination. Thus, for example, the phrase "A, B, C, and/or D" includes A, B, C, and D individually, but also includes any and all combinations and subcombinations of A, B, C, and D.

## Claims

1. A method of making a platform for a solid-state peptide synthesis process, wherein the method comprises:
(i) selecting an insoluble carrier material, wherein the insoluble carrier material is in the form of a plurality of particles;
(ii) coupling a plurality of different linkers to each of the plurality of particles of the insoluble carrier material to form a plurality of platform particles of the platform, wherein the plurality of different linkers comprises:
(a) Fmoc-2,4-dimethoxy-4' -(carboxymethyloxy)-benzhydrylamine (Rink amide linker);
(b) 4-Formyl-3-methoxy-phenoxyacetic acid;
(c) 2-Hydroxy-5-dibenzosuberone;
(d) 4-Hydroxymethylbenzoic acid (HMBA);
(e) 4-Hydroxymethyl-phenoxyacetic acid (HMP linker);
(f) 4-(Fmoc-hydrazino)-benzoic acid;
(g) 4(4-(1-hydroxyethyl)-2-methoxy-5-nitrophenoxy)-butyric acid; and
(h) Fmoc-Suberol (5-Fmoc-amino-2-carboxymethoxy-10,11 -dihydro-5H-dibenzo[a,d] cycloheptene).

2. The method of Claim 1, wherein the insoluble carrier material is a resin material.

3. The method of Claim 1, wherein the particles are microparticles or nanoparticles.

4. A method of making a platform for a solid-state peptide synthesis process, wherein the method comprises:
(i) selecting an insoluble carrier material, wherein the insoluble carrier material is in the form of a plurality of particles;
(ii) coupling a plurality of different linkers to each of the plurality of particles of the insoluble carrier material to form a plurality of platform particles of the platform, wherein the plurality of different linkers comprises at least three of the linkers selected from the group consisting of:
(a) Fmoc-2,4-dimethoxy-4' -(carboxymethyloxy)-benzhydrylamine (Rink amide linker);
(b) 4-Formyl-3-methoxy-phenoxyacetic acid;
(c) 2-Hydroxy-5-dibenzosuberone;
(d) 4-Hydroxymethylbenzoic acid (HMBA);
(e) 4-Hydroxymethyl-phenoxyacetic acid (HMP linker);
(f) 4-(Fmoc-hydrazino)-benzoic acid;
(g) 4(4-(1-hydroxyethyl)-2-methoxy-5-nitrophenoxy)-butyric acid; and
(h) Fmoc-Suberol (5-Fmoc-amino-2-carboxymethoxy-10,11 -dihydro-5H-dibenzo[a,d] cycloheptene).

5. The method of Claim 4, wherein the plurality of different linkers comprises:
(a) Fmoc-2,4-dimethoxy-4' -(carboxymethyloxy)-benzhydrylamine (Rink amide linker);
(b) 4-Formyl-3-methoxy-phenoxyacetic acid; and
(c) 2-Hydroxy-5-dibenzosuberone.

6. The method of Claim 5, wherein the plurality of different linkers further comprises one or more of the linkers selected from the group consisting of:
(d) 4-Hydroxymethylbenzoic acid (HMBA);
(e) 4-Hydroxymethyl-phenoxyacetic acid (HMP linker);
(f) 4-(Fmoc-hydrazino)-benzoic acid;
(g) 4(4-(1-hydroxyethyl)-2-methoxy-5-nitrophenoxy)-butyric acid; and
(h) Fmoc-Suberol (5-Fmoc-amino-2-carboxymethoxy-10,11 -dihydro-5H-dibenzo[a,d] cycloheptene).

7. The method of any one of Claims 4 to 6, wherein the insoluble carrier material is a resin material.

8. The method of any one of Claims 4 to 7, wherein the particles are microparticles or nanoparticles.

9. A platform composition capable of being used in a solid-state peptide synthesis process to synthesize peptides, wherein the platform composition comprises:
(i) an insoluble carrier material, wherein the insoluble carrier material is in the form of a plurality of particles;
(ii) a plurality of different linkers, wherein each of the plurality of particles of the insoluble carrier material is coupled to at least one of each of the different linkers in the plurality of linkers, and wherein the plurality of different linkers comprises:
(a) Fmoc-2,4-dimethoxy-4' -(carboxymethyloxy)-benzhydrylamine (Rink amide linker);
(b) 4-Formyl-3-methoxy-phenoxyacetic acid;
(c) 2-Hydroxy-5-dibenzosuberone;
(d) 4-Hydroxymethylbenzoic acid (HMBA);
(e) 4-Hydroxymethyl-phenoxyacetic acid (HMP linker);
(f) 4-(Fmoc-hydrazino)-benzoic acid;
(g) 4(4-(1-hydroxyethyl)-2-methoxy-5-nitrophenoxy)-butyric acid; and
(h) Fmoc-Suberol (5-Fmoc-amino-2-carboxymethoxy-10,11 -dihydro-5H-dibenzo[a,d] cycloheptene).

10. The platform composition of Claim 9, wherein the insoluble carrier material is a resin material.

11. The platform composition of any one of Claims 9 to 10, wherein the particles are microparticles or nanoparticles.

12. A method comprising:
(a) loading a platform composition of any one of Claims 9 to 11 into a reaction vessel of a solid-phase peptide synthesis synthesizer; and
(b) utilizing the solid-phase peptide synthesis synthesizer to synthesize a first peptide using the platform composition as the platform for peptide synthesis.

13. The method of Claim 12, further comprising:
(c) after the step of synthesizing the first peptide, utilizing the solid-phase peptide synthesis synthesizer to synthesize a second peptide, wherein
(i) the solid-state peptide synthesis synthesizer uses the platform composition as the platform for peptide synthesis and
(ii) the second peptide is a different peptide from the first peptide.

14. The method of any one of Claims 12 to 13, wherein the solid-phase peptide synthesis synthesizer is a continuous flow peptide synthesizer.

15. The method of any one of Claims 12-14, further comprising measuring the flow in the continuous flow peptide synthesizer to quantitatively measure the synthesis of the first peptide.

## Patentansprüche

1. Verfahren zur Herstellung einer Plattform für ein Peptid-Syntheseverfahren im festen Zustand, wobei das Verfahren umfasst:
(i) Auswahl eines unlöslichen Trägermaterials, wobei das unlösliche Trägermaterial in Form einer Vielzahl von Partikeln vorliegt;
(ii) Koppeln einer Vielzahl von verschiedenen Linkern an jedes der Vielzahl von Teilchen des unlöslichen Trägermaterials, um eine Vielzahl von Plattformteilchen der Plattform zu bilden, wobei die Vielzahl von verschiedenen Linkern umfasst:
(a) Fmoc-2,4-Dimethoxy-4'-(carboxymethyloxy)-benzhydrylamin (Rink-Amid-Linker);
(b) 4-Formyl-3-methoxy-phenoxyessigsäure;
(c) 2-Hydroxy-5-dibenzosuberon;
(d) 4-Hydroxymethylbenzoesäure (HMBA);
(e) 4-Hydroxymethyl-phenoxyessigsäure (HMP-Linker);
(f) 4-(Fmoc-Hydrazino)-benzoesäure;
(g) 4(4-(1-Hydroxyethyl)-2-methoxy-5-nitrophenoxy)-buttersäure; und
(h) Fmoc-Suberol (5-Fmoc-Amino-2-carboxymethoxy-10,11-dihydro-5H-dibenzo[a,d]cycloheptene).

2. Verfahren nach Anspruch 1, wobei das unlösliche Trägermaterial ein Harzmaterial ist.

3. Verfahren nach Anspruch 1, wobei die Partikel Mikropartikel oder Nanopartikel sind.

4. Verfahren zur Herstellung einer Plattform für ein Peptidsyntheseverfahren in festem Zustand, wobei das Verfahren umfasst:
(i) Auswahl eines unlöslichen Trägermaterials, wobei das unlösliche Trägermaterial in Form einer Vielzahl von Partikeln vorliegt;
(ii) Koppeln einer Vielzahl von verschiedenen Linkern an jedes der Vielzahl von Teilchen des unlöslichen Trägermaterials, um eine Vielzahl von Plattformteilchen der Plattform zu bilden, wobei die Vielzahl von verschiedenen Linkern mindestens drei der Linker umfasst, die aus der Gruppe ausgewählt sind, die aus den Folgenden besteht:
(a) Fmoc-2,4-Dimethoxy-4'-(carboxymethyloxy)-benzhydrylamin (Rink-Amid-Linker);
(b) 4-Formyl-3-methoxy-phenoxyessigsäure;
(c) 2-Hydroxy-5-dibenzosuberon;
(d) 4-Hydroxymethylbenzoesäure (HMBA);
(e) 4-Hydroxymethyl-Phenoxyessigsäure (HMP-Linker);
(f) 4-(Fmoc-Hydrazino)-benzoesäure;
(g) 4(4-(1-Hydroxyethyl)-2-methoxy-5-nitrophenoxy)-buttersäure; und
(h) Fmoc-Suberol (5-Fmoc-Amino-2-carboxymethoxy-10,11-dihydro-5H-dibenzo[a,d]cycloheptene).

5. Verfahren nach Anspruch 4, wobei die Vielzahl von verschiedenen Linkern umfasst:
(a) Fmoc-2,4-Dimethoxy-4'-(carboxymethyloxy)-benzhydrylamin (Rink-Amid-Linker);
(b) 4-Formyl-3-methoxy-phenoxyessigsäure; und
(c) 2-Hydroxy-5-dibenzosuberon.

6. Verfahren nach Anspruch 5, wobei die Vielzahl unterschiedlicher Linker ferner einen oder mehrere Linker umfasst, die aus der Gruppe ausgewählt sind, die besteht aus:
(d) 4-Hydroxymethylbenzoesäure (HMBA);
(e) 4-Hydroxymethyl-phenoxyessigsäure (HMP-Linker);
(f) 4-(Fmoc-Hydrazino)-benzoesäure;
(g) 4(4-(1-Hydroxyethyl)-2-methoxy-5-nitrophenoxy)-buttersäure; und
(h) Fmoc-Suberol (5-Fmoc-Amino-2-carboxymethoxy-10,11-dihydro-5H-di-benzo[a,d]cycloheptene).

7. Verfahren nach einem der Ansprüche 4 bis 6, wobei das unlösliche Trägermaterial ein Harzmaterial ist.

8. Verfahren nach einem der Ansprüche 4 bis 7, wobei die Partikel Mikropartikel oder Nanopartikel sind.

9. Plattformzusammensetzung, die in einem Festphasen-Peptidsyntheseverfahren zur Synthese von Peptiden verwendet werden kann, wobei die Plattformzusammensetzung umfasst:
(i) ein unlösliches Trägermaterial, wobei das unlösliche Trägermaterial in Form einer Vielzahl von Partikeln vorliegt;
(ii) eine Vielzahl von verschiedenen Linkern, wobei jedes der Vielzahl von Teilchen des unlöslichen Trägermaterials an mindestens einen von jedem der verschiedenen Linker in der Vielzahl von Linkern gekoppelt ist, und wobei die Vielzahl von verschiedenen Linkern umfasst:
(a) Fmoc-2,4-Dimethoxy-4'-(carboxymethyloxy)-benzhydrylamin (Rink-Amid-Linker);
(b) 4-Formyl-3-methoxy-phenoxyessigsäure;
(c) 2-Hydroxy-5-dibenzosuberon;
(d) 4-Hydroxymethylbenzoesäure (HMBA);
(e) 4-Hydroxymethyl-Phenoxyessigsäure (HMP-Linker);
(f) 4-(Fmoc-Hydrazino)-benzoesäure;
(g) 4(4-(1-Hydroxyethyl)-2-methoxy-5-nitrophenoxy)-buttersäure; und
(h) Fmoc-Suberol (5-Fmoc-Amino-2-carboxymethoxy-10,11-dihydro-5H-dibenzo[a,d]cycloheptene).

10. Plattformzusammensetzung nach Anspruch 9, wobei das unlösliche Trägermaterial ein Harzmaterial ist.

11. Plattformzusammensetzung nach einem der Ansprüche 9 bis 10, wobei die Partikel Mikropartikel oder Nanopartikel sind.

12. Ein Verfahren, das Folgendes umfasst:
(a) Laden einer Plattformzusammensetzung nach einem der Ansprüche 9 bis 11 in ein Reaktionsgefäß eines Festphasen-Peptidsynthese-Synthesizers; und
(b) Verwendung des Festphasen-Peptidsynthese-Synthesizers zur Synthese eines ersten Peptids unter Verwendung der Plattformzusammensetzung als Plattform für die Peptidsynthese.

13. Das Verfahren nach Anspruch 12, welches ferner umfasst:
(c) nach dem Schritt der Synthese des ersten Peptids die Verwendung des Festphasen-Peptidsynthese-Synthesizers zur Synthese eines zweiten Peptids, wobei
(i) der Festkörper-Peptidsynthese-Synthesizer die Plattformzusammensetzung als Plattform für die Peptidsynthese verwendet und
(ii) das zweite Peptid ein anderes Peptid als das erste Peptid ist.

14. Verfahren nach einem der Ansprüche 12 bis 13, wobei der Festphasen-Peptidsynthese-Synthesizer ein Peptidsynthesizer mit kontinuierlichem Fluss ist.

15. Verfahren nach einem der Ansprüche 12 bis 14, ferner umfassend das Messen des Flusses in dem Peptidsynthesizer mit kontinuierlichem Fluss, um die Synthese des ersten Peptids quantitativ zu messen.

## Revendications

1. Méthode de fabrication d'une plate-forme pour un processus de synthèse de peptides à l'état solide, dans laquelle la méthode comprend :
(i) sélectionner un matériau de support insoluble, dans lequel le matériau de support insoluble se présente sous la forme d'une pluralité de particules ;
(ii) coupler une pluralité de linkers différents à chacune de la pluralité de particules du matériau de support insoluble pour former une pluralité de particules de plate-forme de la plate-forme, dans laquelle la pluralité de linkers différents comprend au moins trois des linkers choisis dans le groupe constitué de :
(a) Fmoc-2,4-diméthoxy-4'-(carboxyméthyloxy)-benzhydrylamine (linker d'amide de Rink) ;
(b) Acide 4-Formyl-3-méthoxy-phénoxyacétique ;
(c) 2-Hydroxy-5-dibenzosubérone ;
(d) Acide 4-hydroxyméthylbenzoïque (HMBA) ;
(e) Acide 4-hydroxyméthyl-phénoxyacétique (linker de HMP) ;
(f) Acide 4-(Fmoc-hydrazino)-benzoïque ;
(g) Acide 4(4-(1-hydroxyéthyl)-2-méthoxy-5-nitrophénoxy)-butyrique ; et
(h) Fmoc-Subérol (5-Fmoc-amino-2-carboxyméthoxy-10,11-dihydro-5H-dibenzo[a,d]cycloheptène).

2. La méthode de la revendication 1, dans laquelle le matériau de support insoluble est un matériau de résine.

3. La méthode de la revendication 1, dans laquelle les particules sont des microparticules ou des nanoparticules.

4. Méthode de fabrication d'une plate-forme pour un processus de synthèse de peptides à l'état solide, dans laquelle la méthode comprend :
(i) sélectionner un matériau de support insoluble, dans lequel le matériau de support insoluble se présente sous la forme d'une pluralité de particules ;
(ii) coupler une pluralité de linkers différents à chacune de la pluralité de particules du matériau de support insoluble pour former une pluralité de particules de plate-forme de la plate-forme, dans laquelle la pluralité de linkers différents comprend au moins trois des linkers choisis dans le groupe constitué de :
(a) Fmoc-2,4-diméthoxy-4'-(carboxyméthyloxy)-benzhydrylamine (linker d'amide de Rink) ;
(b) Acide 4-Formyl-3-méthoxy-phénoxyacétique ;
(c) 2-Hydroxy-5-dibenzosubérone ;
(d) Acide 4-hydroxyméthylbenzoïque (HMBA) ;
(e) Acide 4-hydroxyméthyl-phénoxyacétique (linker de HMP) ;
(f) Acide 4-(Fmoc-hydrazino)-benzoïque ;
(g) Acide 4(4-(1-hydroxyéthyl)-2-méthoxy-5-nitrophénoxy)-butyrique ; et
(h) Fmoc-Subérol (5-Fmoc-amino-2-carboxyméthoxy-10,11-dihydro-5H-dibenzo[a,d] cycloheptène).

5. La méthode de la revendication 4, dans laquelle la pluralité de linkers différents comprend :
(a) Fmoc-2,4-diméthoxy-4'-(carboxyméthyloxy)-benzhydrylamine (linker d'amide de Rink) ;
(b) Acide 4-Formyl-3-méthoxy-phénoxyacétique ; et
(c) 2-Hydroxy-5-dibenzosubérone.

6. La méthode de la revendication 5, dans laquelle la pluralité de linkers différents comprend en outre un ou plusieurs linkers choisis dans le groupe constitué de :
(d) Acide 4-Hydroxyméthylbenzoïque (HMBA) ;
(e) Acide 4-hydroxyméthyl-phénoxyacétique (linker de HMP) ;
(f) Acide 4-(Fmoc-hydrazino)-benzoïque ;
(g) Acide 4(4-(1-hydroxyéthyl)-2-méthoxy-5-nitrophénoxy)-butyrique ; et
(h) Fmoc-Subérol (5-Fmoc-amino-2-carboxyméthoxy-10,11-dihydro-5H-dibenzo[a,d] cycloheptène).

7. La méthode de l'une quelconque des revendications 4 à 6, dans lequel le matériau de support insoluble est un matériau de résine.

8. La méthode de l'une quelconque des revendications 4 à 7, dans laquelle les particules sont des microparticules ou des nanoparticules.

9. Composition de plate-forme pouvant être utilisée dans un processus de synthèse de peptides à l'état solide pour synthétiser des peptides, dans laquelle la composition de plate-forme comprend :
(i) un matériau de support insoluble, dans lequel le matériau de support insoluble se présente sous la forme d'une pluralité de particules ;
(ii) une pluralité de linkers différents, dans laquelle chacune de la pluralité de particules du matériau de support insoluble est couplée à au moins un de chacun de linkers différents dans la pluralité de linkers, et dans laquelle la pluralité de linkers différents comprend :
(a) Fmoc-2,4-diméthoxy-4'-(carboxyméthyloxy)-benzhydrylamine (linker d'amide de Rink) ;
(b) Acide 4-Formyl-3-méthoxy-phénoxyacétique ;
(c) 2-Hydroxy-5-dibenzosubérone ;
(d) Acide 4-hydroxyméthylbenzoïque (HMBA) ;
(e) Acide 4-hydroxyméthyl-phénoxyacétique (linker de HMP) ;
(f) Acide 4-(Fmoc-hydrazino)-benzoïque ;
(g) Acide 4(4-(1-hydroxyéthyl)-2-méthoxy-5-nitrophénoxy)-butyrique ; et
(h) Fmoc-Subérol (5-Fmoc-amino-2-carboxyméthoxy-10,11-dihydro-5H-dibenzo[a,d] cycloheptène).

10. La composition de plate-forme de la revendication 9, dans laquelle le matériau de support insoluble est un matériau de résine.

11. La composition de plate-forme de l'une quelconque des revendications 9 à 10, dans laquelle les particules sont des microparticules ou des nanoparticules.

12. Méthode comprenant :
(a) charger une composition de plate-forme de l'une quelconque des revendications 9 à 11 dans une cuve de réaction d'un synthétiseur de synthèse de peptides en phase solide ; et
(b) utiliser le synthétiseur de synthèse de peptides en phase solide pour synthétiser un premier peptide en utilisant la composition de plate-forme comme plate-forme pour la synthèse de peptides.

13. La méthode de la revendication 12, comprenant en outre :
(c) après l'étape de synthèse du premier peptide, utiliser le synthétiseur de synthèse de peptides en phase solide pour synthétiser un second peptide, dans laquelle
(i) le synthétiseur de synthèse de peptides à l'état solide utilise la composition de plate-forme comme plate-forme pour la synthèse de peptides et
(ii) le second peptide est un peptide différent du premier peptide.

14. La méthode de l'une des revendications 12 à 13, dans laquelle le synthétiseur de synthèse de peptides en phase solide est un synthétiseur de peptides à flux continu.

15. La méthode de l'une quelconque des revendications 12 à 14, comprenant en outre mesurer le flux dans le synthétiseur de peptides à flux continu pour mesurer quantitativement la synthèse du premier peptide.
